# EUROPEAN PATENT APPLICATION

(11) **EP 2 740 447 A1**
(43) Date of publication of application: **11.06.2014**
(21) Application number: 13192458.1
(22) Date of filing: 12.11.2013
(51) Int. Cl.: A61F 9/00

(54) **Contact-lens putting support tool**

(30) Priority: 07.12.2012 JP 2012268707
(71) Applicant: Medi Toreck Co., Ltd., Kanagawa-ken 231-0004 (JP)
(72) Inventor: Saitoh, Kazuko, Kanagawa-ken, 231-0004 (JP); Saitoh, Toshihiko, Kanagawa-ken, 231-0004 (JP)
(74) Representative: HOFFMANN EITLE

(57) **Abstract**

Disclosed is a contact-lens putting support tool (100) including a rod-like handle portion (110) held by fingers of a wearer of a contact lens and a head portion (120) for causing the contact lens to adhere thereto through a liquid to take out the contact lens from a container, the contact lens having a front surface thereof wet with the liquid. A lower part of the handle portion is flat such that the contact-lens putting support tool is softly placed on a flat surface. The head portion is directed obliquely upward with respect to an axis formed by the handle portion, is curved downward, and has a spoon-like shape of which upper and lower surfaces are rounded. A convex surface of the contact lens having a concave surface thereof adhering to the lower surface of the head portion is placed at a predetermined position of a contact-lens putting tool (130).

## Description

### BACKGROUND OF THE INVENTION

### 1. Field of the Invention

The present invention relates to the technique of a support tool used to take out a contact lens from a container without touching the contact lens with a hand and place the taken-out contact lens on a contact-lens putting tool.

### 2. Description of the Related Art

In recent years, there has been an increase in the development of tools used to facilitate the putting and removing operations of contact lenses.

For example, JP 2012-130638 A proposes a contact-lens putting and removing tool that facilitates the putting and removing operations of contact lenses for first-time contact-lens users and persons having problems with the putting and removing operations and puts no burden on eyes.

However, in the above technique, a contact lens is touched by a hand when taken out from a container and placed at a predetermined position on the tool. Therefore, various bacteria adhere to the contact lens, which results in an unsanitary condition.

In addition, there are difficulties in taking out a contact lens from a container with a hand of a wearer himself/herself and placing the contact lens at the predetermined position on the tool if the wearer has long nails or fake fingernails.

In view of the above problems, it is an object of the present invention to provide a contact-lens putting support tool that facilitates the operations of taking out a contact lens from a container and placing the taken-out contact lens on a putting tool and performs the operations in a sanitary way.

### SUMMARY OF THE INVENTION

An embodiment of the present invention provides a contact-lens putting support tool used to take out a contact lens from a container without directly touching the contact lens with a hand and place the contact lens on a contact-lens putting tool used to put the contact lens in an eye without directly touching the contact lens with the hand. The contact-lens putting support tool includes: a rod-like handle portion held by fingers of a wearer of the contact lens to perform an operation; and a head portion for causing the contact lens to adhere thereto through a liquid to take out the contact lens from the container, the contact lens being stored in the container and having a front surface thereof wet with the liquid. A lower part of the handle portion is flat such that the contact-lens putting support tool is softly placed on a flat surface. The head portion is directed obliquely upward with respect to an axis formed by the handle portion, is curved downward, and has a spoon-like shape of which upper and lower surfaces are rounded. A convex surface of the contact lens having a concave surface thereof adhering to the lower surface of the head portion is placed at a predetermined position of the contact-lens putting tool.

Besides, another embodiment of the present invention provides the contact-lens putting support tool described above, wherein the contact-lens putting tool has hand-held shafts, upper ends positioned at one end of the contact-lens putting tool and formed by dividing the contact-lens putting tool into two branches, and a lower end positioned at the other end of the contact-lens putting tool and formed into a rod shape. The upper ends have, at both tips thereof, respective adhesive members for causing the contact lens adhere thereto. The lower end has, at a tip thereof, a trumpet-like member of which an outer shape is widened forward like a trumpet. The trumpet-like member has, at a tip thereof, an opening portion recessed such that a curved surface of the contact lens is mounted in a natural form. The contact-lens putting tool has curved shapes at portions thereof ranging from the hand-held shafts to the upper ends and has curved shapes at portions thereof ranging from the hand-held shafts to the lower end. The opening portion is directed in a direction in which the contact-lens putting tool is curved from the hand-held shafts to the lower end. The contact-lens putting tool removes the contact lens from the eye by holding the contact lens put in the eye with the adhesive members provided at both the tips of the upper ends. The contact-lens putting tool puts the contact lens in the eye, the contact lens being placed on the opening portion with an eye-contacting surface thereof directed outward. A predetermined position of the contact-lens putting tool is the opening portion of the trumpet-like member.

Besides, a still another embodiment of the present invention provides the contact-lens putting support tool described above, wherein the contact-lens putting support tool is made of silicon.

With a contact-lens putting support tool disclosed herein, it is possible to facilitate the operations of taking out a contact lens from a container and placing the taken-out contact lens on a putting tool and perform the operations in a sanitary way.

Other objects, features and advantages of the present invention will become more apparent from the following detailed description when read in conjunction with the accompanying drawings.

### BRIEF DESCRIPTION OF THE DRAWINGS

FIG. 1 is a perspective view of a contact-lens putting support tool according to an embodiment;
FIG. 2 is a side view of the contact-lens putting support tool according to the embodiment;
FIG. 3 is a cross-sectional view of the contact-lens putting support tool according to the embodiment;
FIG. 4 is a perspective view of a contact-lens putting tool according to the embodiment;
FIG. 5 is a cross-sectional view of the contact-lens putting tool according to the embodiment;
FIG. 6 is a view showing an example of a contact lens stored in a container;
FIG. 7 is a view showing a state in which the contact lens is caused to adhere to the contact-lens putting support tool according to the embodiment; and
FIG. 8 is a view showing an example of the use state of the contact-lens putting tool according to the embodiment.

### DETAILED DESCRIPTION OF THE PREFERRED EMBODIMENT

An embodiment for carrying out the present invention will be described with reference to the accompanying drawings.

### (Structure of Contact-Lens Putting Support Tool According to Embodiment)

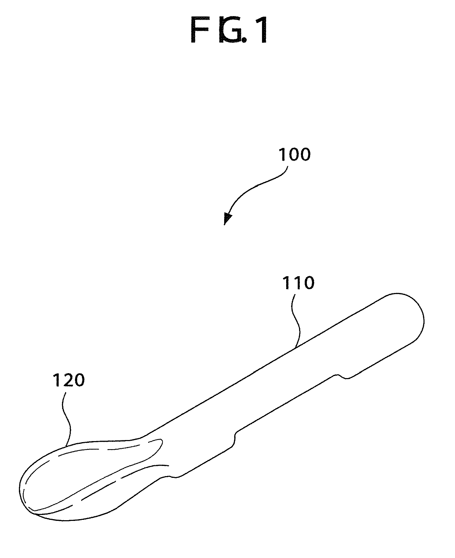

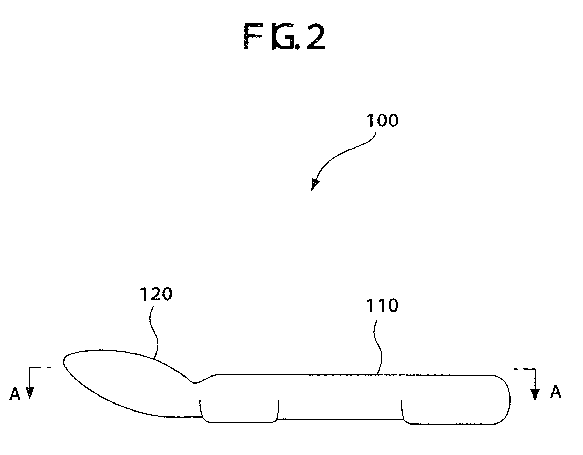

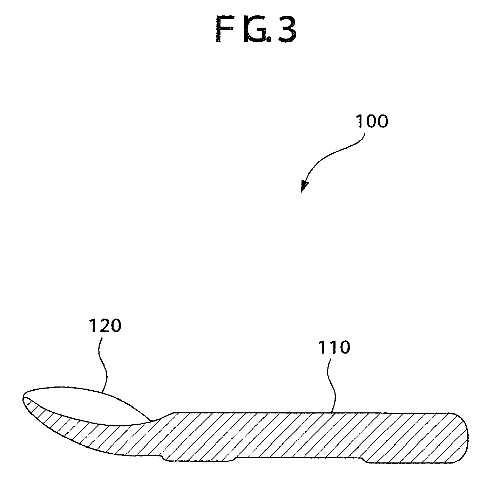

A description will be given, with reference to FIGS. 1 to 3, of a contact-lens putting support tool (hereinafter simply referred to as a "putting support tool") 100 according to the embodiment. FIG. 1 is a perspective view for explaining the general configuration of the putting support tool 100. FIGS. 2 and 3 are a side view and a cross-sectional view for explaining the characteristics of the putting support tool 100.

As shown in FIG. 1, the putting support tool 100 has a handle portion 110 and a head portion 120. The handle portion 110 is a portion held by the fingers of a wearer 160 of a contact lens 150 to perform an operation and has a rod-like shape. In addition, the putting support tool 100 is preferably made of silicon from a sanitary standpoint.

As shown in FIGS. 2 and 3, the lower part of the handle portion 110 is flat such that the putting support tool 100 keeps its stable posture when placed on a flat surface. Note that the lower part of the handle portion 110 may be flat entirely or only part of the handle portion 110 contacting a flat surface may be flat. With such a shape of the head portion 110, the posture of the putting support tool 100 can be stabilized in a state in which the head portion 120 is not in contact with unsanitary matter when the putting support tool 100 is not in use.

As shown in FIGS. 2 and 3, the head portion 120 is directed obliquely upward with respect to an axis formed by the rod-like handle portion 110. With the combination of such a shape of the head portion 120 and the shape of the lower part of the handle portion 110, the head portion 120 can be kept in a state so as not to be in contact with unsanitary matter when the putting support tool 100 is not in use. In addition, such a shape of the head portion 120 contributes to the easiness of an operation in a state in which the handle portion 110 is held.

Moreover, the head portion 120 is curved downward and has a spoon-like shape of which the upper and lower surfaces are rounded. With such a shape of the head portion 120, the contact lens 150 can be taken out from a container 140 without touching the contact lens 150 with a hand.

The contact lens 150 is taken out from the container 140 in such a way that the rounded lower surface of the head portion 120 is caused to adhere to the contact lens 150 through a liquid, the contact lens 150 being stored in the container 140 and having the front surface thereof wet with the liquid. Here, the liquid is a liquid for cleaning and preserving the contact lens 150, water, or the like. Note that since the contact lens 150 is normally curved and has convex and concave surfaces, it is suitable to take out the contact lens 150 from the container 140 with the concave surface of the contact lens 150 adhering to the lower surface (convex surface) of the head portion 120 as a method of using the putting support tool 100. However, as a method of using the putting support tool 100, it may also be possible to take out the contact lens 150 from the container 140 with the contact lens 150 adhering to or placed on the upper surface (concave surface) of the head portion 120.

### (Structure of Contact-Lens Putting Tool According to Embodiment)

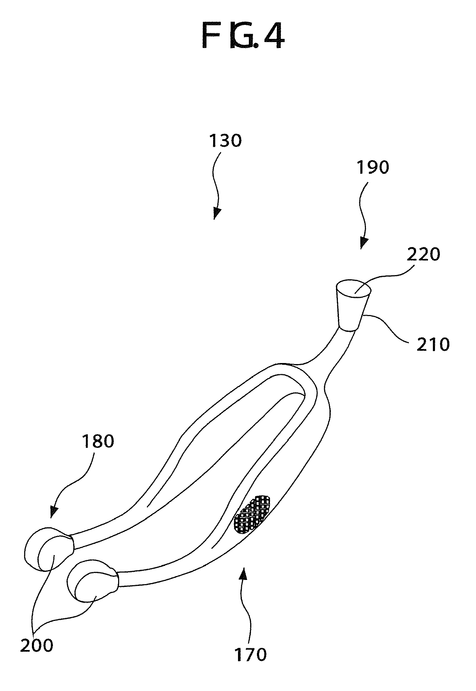

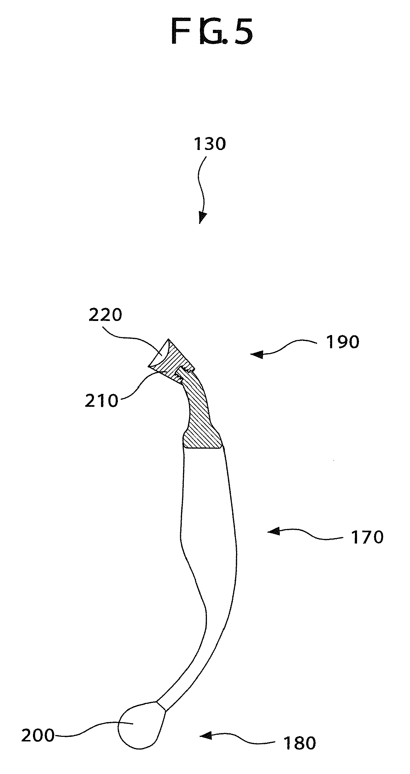

Next, a description will be given, with reference to FIGS. 4 and 5, of a contact-lens putting tool (hereinafter simply referred to as a "putting tool") 130 according to the embodiment. FIG. 4 is a perspective view for explaining the general configuration of the putting tool 130. FIG. 5 is a cross-sectional view for explaining the characteristics of the putting tool 130.

The putting tool 130 is a contact lens putting/removing tool used to easily and cleanly put and remove the contact lens 150 in and from an eye. As shown in FIGS. 4 and 5, the putting tool 130 has hand-held shafts 170, upper ends 180, and a lower end 190.

The upper ends 180 are positioned at one end of the putting tool 130 and formed by dividing the putting tool 130 into two branches. The upper ends 180 have, at both the tips thereof, respective adhesive members 200 for causing the contact lens 150 to adhere thereto or holding the same therebetween.

The lower end 190 is positioned at the other end of the putting tool 130 and formed into a rod shape. The lower end 190 has, at the tip thereof, a trumpet-like member 210 of which the outer shape is widened forward like a trumpet. The trumpet-like member 210 has, at the tip thereof, an opening portion 220 recessed such that the curved surface of the contact lens 150 is mounted in a natural form.

The putting tool 130 has curved shapes at the portions thereof ranging from the hand-held shafts 170 to the upper ends 180 and has curved shapes at the portions thereof ranging from the hand-held shafts 170 to the lower end 190.

The opening portion 220 is directed in a direction in which the putting tool 130 is curved from the hand-held shafts 170 to the lower end 190.

The putting tool 130 removes the contact lens 150 from the eye by holding the contact lens 150 put in the eye with the adhesive members 200 provided at both tips of the upper ends 180. In addition, the putting tool 130 has the contact lens 150 placed on the opening portion 220 with the eye-contacting surface (concave surface) of the contact lens 150 directed outward. The contact lens 150 is moved close to the eye together with the putting tool 130 (the opening portion 220) to be put in the eye.

The putting support tool 100 takes out the contact lens 150 from the container 140 with the concave surface of the curved contact lens 150 adhering to the lower surface of the head portion 120, and places the convex surface (surface not adhering to the head portion 120) of the taken-out contact lens 150 on the recessed part of the opening portion 220.

### (Method of Using Contact-Lens Putting Support Tool According to Embodiment)

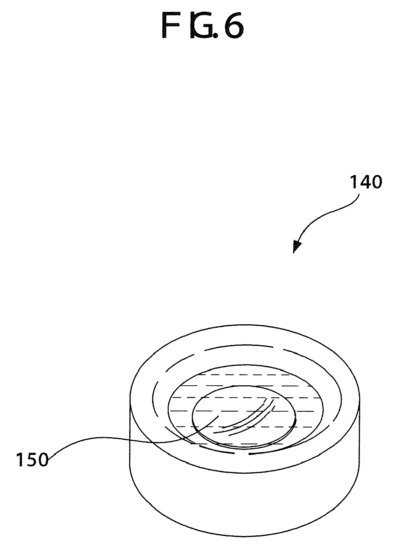

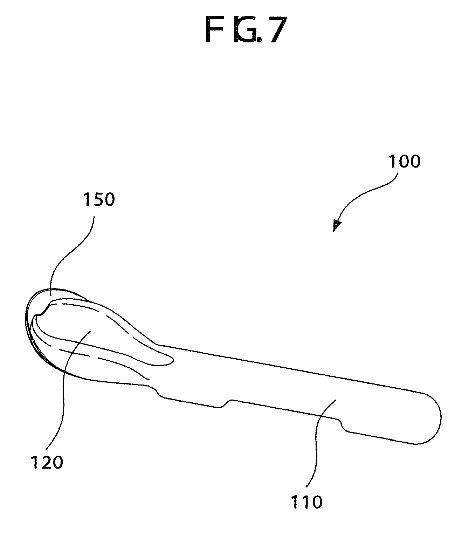

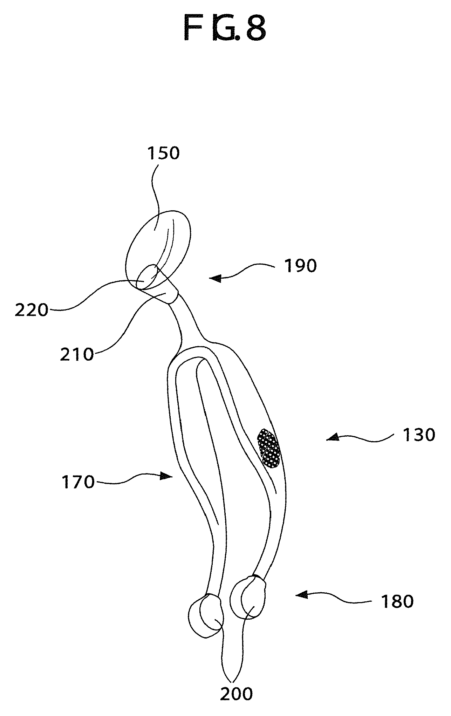

Next, a description will be given, with reference to FIGS. 6 to 8, of a method of using the putting support tool 100. FIG. 6 is a view showing a mode of the contact lens 150 stored in the container 140. FIG. 7 is a view showing a state in which the contact lens 150 is caused to adhere to the putting support tool 100. FIG. 8 is a view showing a state in which the contact lens 150 is placed on the putting tool 130 by the putting support tool 100.

As shown in FIG. 6, the contact lens 150 is stored in the container 140 in a state of floating in a liquid.

As a first step, the wearer 160 puts the head portion 120 of the putting support tool 100 in the liquid of the container 140.

Next, as a second step, the wearer 160 moves the lower surface of the head portion 120 close to the concave surface of the contact lens 150 floating in the liquid to cause the contact lens 150 to adhere to the lower surface and removes the contact lens 150 from the container 140. As a result, as shown in FIG. 7, the contact lens 150 removed from the container 140 is in a state of adhering to the lower surface of the head portion 120.

As a third step, the wearer 160 places the contact lens 150 adhering to the lower surface of the head portion 120 on the recessed part of the opening portion 220 of the putting tool 130 so as to bring the convex surface (outside surface) thereof into contact with the recessed part. As a result, as shown in FIG. 8, the contact lens 150 is placed on the putting tool 130 with the eye-contacting concave surface thereof directed outward.

Finally, as a fourth step, the wearer 160 moves the contact lens 150 placed on the opening portion 220 close to the eye and puts the same in the eye.

Note that it may also be possible for the wearer 160 to take out the contact lens 150 from the container 140 through the upper surface of the head portion 120 and directly put the taken-out contact lens 150 in the eye without using the putting tool 130 as a method of putting the contact lens 150.

## Claims

1. A contact-lens putting support tool used to take out a contact lens from a container without directly touching the contact lens with a hand and place the contact lens on a contact-lens putting tool used to put the contact lens in an eye without directly touching the contact lens with the hand, the contact-lens putting support tool comprising:
a rod-like handle portion held by fingers of a wearer of the contact lens to perform an operation; and
a head portion for causing the contact lens to adhere thereto through a liquid to take out the contact lens from the container, the contact lens being stored in the container and having a front surface thereof wet with the liquid, wherein
a lower part of the handle portion is flat such that the contact-lens putting support tool is softly placed on a flat surface,
the head portion is directed obliquely upward with respect to an axis formed by the handle portion, is curved downward, and has a spoon-like shape of which upper and lower surfaces are rounded, and
a convex surface of the contact lens having a concave surface thereof adhering to the lower surface of the head portion is placed at a predetermined position of the contact-lens putting tool.

2. The contact-lens putting support tool according to claim 1, wherein
the contact-lens putting tool has
hand-held shafts,
upper ends positioned at one end of the contact-lens putting tool and formed by dividing the contact-lens putting tool into two branches, and
a lower end positioned at the other end of the contact-lens putting tool and formed into a rod shape,
the upper ends have, at both tips thereof, respective adhesive members for causing the contact lens adhere thereto,
the lower end has, at a tip thereof, a trumpet-like member of which an outer shape is widened forward like a trumpet,
the trumpet-like member has, at a tip thereof, an opening portion recessed such that a curved surface of the contact lens is mounted in a natural form,
the contact-lens putting tool has curved shapes at portions thereof ranging from the hand-held shafts to the upper ends and has curved shapes at portions thereof ranging from the hand-held shafts to the lower end,
the opening portion is directed in a direction in which the contact-lens putting tool is curved from the hand-held shafts to the lower end,
the contact-lens putting tool removes the contact lens from the eye by holding the contact lens put in the eye with the adhesive members provided at both the tips of the upper ends,
the contact-lens putting tool puts the contact lens in the eye, the contact lens being placed on the opening portion with an eye-contacting surface thereof directed outward, and
a predetermined position of the contact-lens putting tool is the opening portion of the trumpet-like member.

3. The contact-lens putting support tool according to claim 1 or 2, wherein
the contact-lens putting support tool is made of silicon.
